# EUROPEAN PATENT SPECIFICATION

(11) **EP 2 529 700 B1**
(45) Date of publication and mention of the grant of the patent: **14.09.2016**
(21) Application number: 12169974.8
(22) Date of filing: 30.05.2012
(51) Int. Cl.: A61F 2/16

(54) **Intraocular lens injection instrument**
Injektor für Intraokularlinsen
Injecteur pour lentilles intraoculaires

(30) Priority: 31.05.2011 JP 2011122446
(43) Date of publication of application: 05.12.2012
(73) Proprietor: Nidek Co., Ltd., Gamagori-shi, Aichi 443-0038 (JP)
(72) Inventor: Nagasaka, Shinji, Gamagori-shi, Aichi 4430038 (JP)
(74) Representative: Prüfer & Partner mbB Patentanwälte · Rechtsanwälte

(56) References cited:
- EP-A1- 2 255 751
- US-A1- 2003 212 407
- US-A1- 2009 043 313
- US-A1- 2011 046 635

## Description

### BACKGROUND OF THE INVENTION

### Field of the Invention

The present invention relates to an intraocular lens injection instrument for injecting an intraocular lens into an eye.

### Related Art according to US2003/212407 A1

A foldable intraocular lens (IOL) is folded into a tiny piece and then injected in an eye by use of an injector. One example of the injector is configured to store an IOL in the injector with a holding member without applying stress to the IOL. In use, the holding member is detached to release the IOL from the holding or retention state. Another example is an injector configured to store an IOL outside a push rod. In use, a predetermined pressing part presses the IOL onto a push axis to release the IOL from a holding state (US2003/0212407A1).

An intraocular lens injection instrument comprising a restriction member attached to the cylinder body part to restrict movement of the IOL outside the push axis without applying stress to the IOL during storage of the IOL is disclosed in document EP-A-2 255 751.

### SUMMARY OF INVENTION

### Problems to be Solved by the Invention

However, most foldable IOLs have viscosity depending on characteristics of their materials. Therefore, some IOLs may stick to an inner wall of an injector outside a push axis when the IOL is stored inside the injector for a long term. When the IOL sticking to the inner wall of the injector outside the push axis is pressed in one stroke onto the push axis by a predetermined pressing part, the IOL cannot be unstuck appropriately due to the differences in local sticking state. Thus, the IOL cannot be arranged correctly on the push axis.

The present invention is made to solve the above problem of prior arts and has a purpose to provide an intraocular lens injection instrument capable of ideally placing an IOL stored outside a push axis onto the axis.

### Means of Solving the Problems

To solve the above problem, an intraocular lens injection instrument according to claim 1 is provided.

Further developments of the present invention are given in the dependent claims.

### BRIEF DESCRIPTION OF THE DRAWINGS

FIG. 1 is an external schematic view of an injector;
FIG. 2 is a schematic sectional view of the injector;
FIG. 3A is an enlarged sectional view of an end portion of the injector and its surroundings during storage;
FIG. 3B is an enlarged sectional view of the end portion of the injector and its surroundings during use;

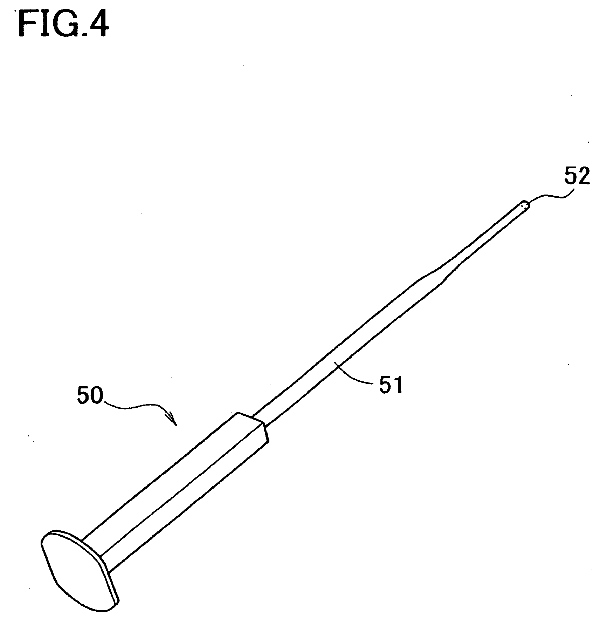

FIG. 4 is an explanatory view of a configuration of a plunger;
FIG. 5A is an enlarged sectional view of an end portion of an injector and its surroundings during use in a second embodiment; and
FIG. 5B is a sectional view of a pressing part in a modification of the second embodiment.

### DESCRIPTION OF EMBODIMENTS

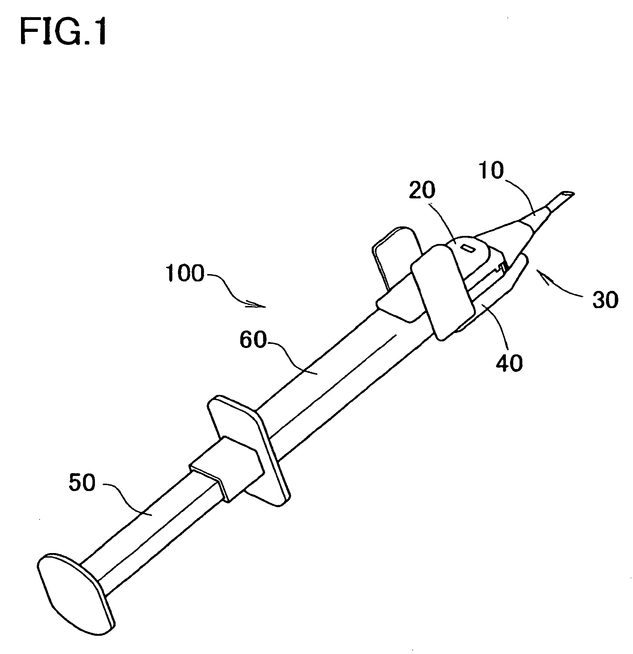

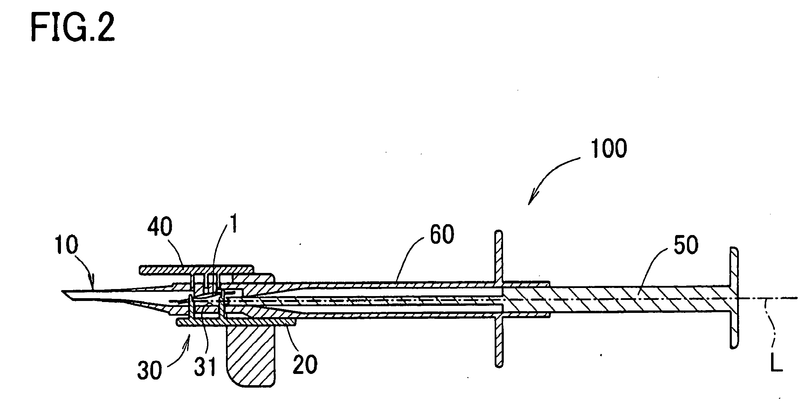

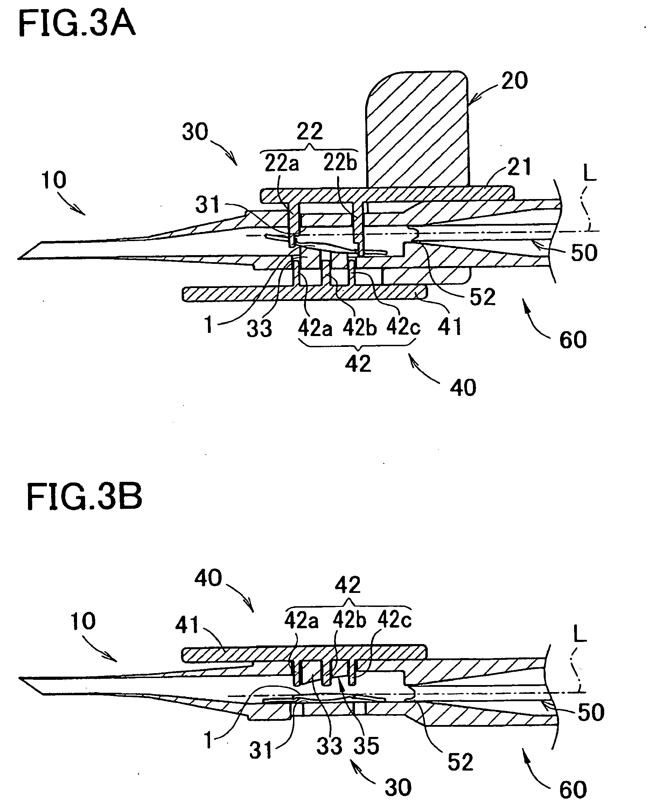

Detailed description of the present invention will be now explained accompanied by the drawings. FIG. 1 is an external schematic view of an injector 100. FIG. 2 is a sectional view of the injector 100. FIGs. 3A and 3B are enlarged sectional views of an end portion of the injector 100 and its surroundings.

The injector 100 includes an injection part 10, a restriction member 20, a setting part 30, a press member 40, a push member (hereinafter, referred to as a plunger) 50, and a cylinder body part 60. The hollow injection part 10 is provided in a front end of the cylinder body part 60 with a region (an inner wall shape) of which an inner diameter gradually decreases (tapers) toward its front end. A soft intraocular lens (IOL) 1 is folded into a tiny piece through a hollow portion of the injection part 10 and injected outside. The above mentioned injector 100 is a resin-molded disposable type.

During storage and conveyance, the injector 100 of the present embodiment is oriented with the restriction member 20 placed above the press member 40 (refer to FIG. 1). On the other hand, during use, the injector 100 is turned upside down from the state in FIG. 1 so that the press member 40 is located above the restriction member 20 (refer to FIG. 2).

The setting part 30 is provided in a rear end of the injection part 10 to hold the IOL 1 released from a holding state by the restriction member 20 (the IOL 1 is disposed on a push axis L)

Incidentally, the state "the IOL 1 lies on the push axis" in the present embodiment includes both cases that a periphery (edge) of the IOL 1 coincides with the push axis L of the plunger 50 and that the IOL 1 is displaced from the axis L to an extent that the IOL 1 can be pushed out by the plunger 50.

The setting part 30 has an upper part designed to be opened and closed with a known configuration such as a hinge, which is not shown in the figure. Thereby, the IOL 1 can be placed inside the injector 100 from the opened upper part of the setting part 30. The setting part 30 is closed again after the IOL 1 is placed inside the injector 100, thus the IOL 1 is stored in the injector 100.

As the IOL 1, known IOLs foldable with use of an injector are selectable, such as a three-piece type IOL having an optical part attached with a pair of loop-shaped support parts and a one-piece type IOL in which support parts and an optical part are integrally formed.

The setting part 30 is formed with a plurality of through holes, which are not assigned with reference signs in the figure, formed to extend perpendicular to the push axis L of the plunger 50, and the restriction member 20 is attached to the cylinder body part 60 through these through holes. A ceiling portion 33 is provided above a setting surface 31 on which the IOL 1 is to be disposed when using, and formed with a setting surface 35 on which the IOL 1 is to be disposed when storing. Thus, a space (clearance) for storing the IOL 1 is created between the setting surfaces 31 and 35. Further, the ceiling portion 33 (setting surface 35) is formed with a plurality of through holes omitted with the reference signs, formed to extend perpendicular to the axis L so that the press member 40 is attached to an opposite side of the cylinder body part 60 through these through holes to face the restriction member 20.

The setting surface 35 has a predetermined slope (angle) with respect to the push axis L (refer to FIGs. 3A and 3B). In the present embodiment, the setting surface 35 is slanted so as to be gradually (successively) away from the axis L from the front end toward the rear end of the injector 100 (from a left end to a right end in FIGs. 3A and 3B).

As an alternative example, the setting surface 35 may be formed to slope from the rear end to the front end so as to be gradually (successively) away from the push axis L. In addition, the height (length) of the setting surface 35 is determined to keep the IOL 1 placed on the surface 35 from lying on or obstructing the axis L.

When the setting surface 35 is formed with a slope, a partial peripheral edge of the optical part of the IOL 1 (a part closer to the front end of the injector 100) is placed and held more closer to the push axis L. Thereby, displacement and unintended deformation of the IOL 1 hardly occurs when the IOL 1 is moved from outside onto the push axis L.

The restriction member 20 is to restrict movement of the IOL 1 during storage and conveyance of the injector 100 between the setting surface 35 and the ceiling portion 33 without causing stress on the IOL 1. The restriction member 20 includes a base 21 which is formed with two restriction parts 22 (22a, 22b) arranged facing each other in a front and rear direction (in a direction of the push axis). A distance between the restriction parts 22a and 22b is almost equal to a diameter of the optical part of the IOL 1. Thus, the IOL 1 placed on the setting surface 35 is prevented from moving (displacement) in the front and rear direction.

The restriction parts 22a and 22b are of a pillar shape, each extending downward by a predetermined length from the base 21, and each height (length) of the parts 22a and 22b is made almost equal to a vertical position of the peripheral edge of the optical part of the IOL 1 that is placed on the setting surface 35. In accordance with the configuration that the setting surface 35 is formed to slope with respect to the push axis L, the restriction part 22b is formed longer (higher) than the restriction part 22a. Thereby, the IOL 1 is prevented from moving in a horizontal direction (front-rear and left-right directions) by each inner wall of the restriction parts 22a and 22b.

Further, distal ends of the restriction part 22 are formed with cutouts (omitted with reference signs in the figure) formed to conform to the shape of the optical part. These cutouts are positioned to fit the peripheral edge of the optical part, so that the IOL 1 is prevented from moving in the vertical direction. The restriction member 20 is attached to the cylinder body part 60 in a manner that the distal ends of the restriction part 22 are inserted in the through holes of the setting surface 31.

The press member 40 is to release the IOL 1 from the holding state outside the push axis L and push the IOL 1 onto the axis L. The press member 40 is configured with a base 41 and press portions (herein, three press portions 42a to 42c) formed on the base 41. Each press portion 42a to 42c of pillar shape, extending from the base 41, is formed in positions corresponding to the peripheral edge and a center of the optical part of the IOL 1 when the IOL 1 is placed on the setting surface 35. The length of each press portion 42a to 42c is determined in a manner that each end of the press portion 42a to 42c comes to contact with the IOL 1 (optical part) when the press portions 42a to 42c are inserted in the through holes formed in the ceiling portion 33 side, but each end does not contact or touch the plunger 50 moved back and forth on the axis L even when the press member 40 is fully pressed.

The IOL 1 is pressed by the above-mentioned press member 40 to release the restriction (holding) of the IOL 1 by the restriction part 22. The press portion 42 has such a length as not to reach the axis L and thus does not obstruct the back and forth movement of the plunger 50. Accordingly, the push-out operation of the IOL 1 by the plunger 50 can be smoothly conducted without detaching the press member 40.

While the press portions 42a to 42c have the equal length (height), the setting surface 35 is slanted. For this reason, the press portions 42a to 42c successively come into contact with the optical part of the IOL 1 from the rear end side of the injector 100. Thus, by changing timing of contact between the optical part and each press portion 42a to 42c, the optical part as a whole is not pushed out in one stroke but pushed out (moved) in order in accordance with the pressing amount of the press member 40 even if the IOL 1 remains sticking to the setting surface 35 during the storage.

In other words, a contact region of the press portion 42 with the IOL 1 (optical part) increases from one side of the peripheral edge of the optical part to the opposite side of the peripheral edge (in one direction), and thus the IOL 1 sticking to the setting surface 35 is successively peeled off from the one side. As a result, combination of the press member 40 and the setting surface 35 of the present embodiment forms pressing means for successively pressing the optical part of the IOL.

The plunger 50 includes a push rod 51 to be moved back and forth in an axial direction inside the cylinder body part 60 and a tip 52 to be brought into contact with the IOL 1 as a distal end of the push rod 51. From a state that the tip 52 is in contact with the IOL 1, the IOL 1 is pushed toward a front end of the cylinder body part 60 by the stress applied by the push rod 51, and thereby the IOL 1 is folded into a tiny piece and injected in the eye. In the present embodiment, the tip 52 has a concave shape so as to hold or catch the edge of the optical part of the IOL 1.

For storing the IOL 1 in the above-mentioned injector 100, the injector 100 is placed first in an orientation as shown in FIG. 1 and the restriction member 20 is detached. Then, the setting part 30 is opened by a not-shown dividing member so that the ceiling portion 33 appears and the IOL 1 is placed on the setting surface 35. After that, the setting part 30 is closed. Subsequently, the restriction parts 22a and 22b of the restriction member 20 are inserted in the through holes of the setting part 30 to attach the restriction member 20 to the cylinder body part 60 again. Incidentally, during this operation, the push rod 51 of the plunger 50 is pulled backward (in a lower left direction in FIG. 1) so that the tip 52 does not exist in the space where the IOL 1 is placed. In a state that the IOL 1 is set in the injector 100 as mentioned above, the injector 100 is wrapped and sterilized for storage.

The following explanation is made to explain a push-out operation to inject the IOL 1 in the eye by use of the injector 100 stored inside with the IOL 1. While the IOL 1 is stored in the injector 100 as shown in FIG. 3A, movement of the IOL 1 in the horizontal direction is restricted by the restriction parts 22a and 22b. Movement of the IOL 1 in the vertical direction is also restricted by the cutouts formed in the restriction parts 22a, 22b. A clearance between each restriction part 22a, 22b and the setting surface 35 is formed as narrow as possible, so that the IOL 1 hardly moves during the storage and conveyance. Further, since no stress acts on the IOL 1 during the storage and conveyance, the lens is hard to be deformed under a long-term storage condition.

When the above-mentioned injector 100 is to be used, as shown in FIG. 3B, the cylinder body part 60 is placed upside down (so that the setting surface 35 is placed above the setting surface 31). Then, the restriction member 20 is pulled out of the cylinder body part 60 to release the movement restriction of the IOL 1. A known viscoelastic substance (or perfusion liquid) is applied in the cylinder body part 60 through a not-shown small hole provided in the cylinder body part 60 (for example, near the setting part 30). Even though the through holes of the setting surface 31 appear when the restriction member 20 is detached, the opening diameter of each through hole is determined to be small enough so as not to leak or to hardly leak the viscoelastic substance (or perfusion liquid). Alternatively, a lid member, which is not shown in the figure, to cover the through holes may be attached.

Depending on a storage condition (storage term) of the IOL 1, there is a case that the optical part of the IOL 1 remains sticking to the setting surface 35 due to viscosity of the IOL 1 even after the restriction member 20 is detached. If the overall IOL 1 sticking to the setting surface 35 is pressed in one stroke, the peripheral edge of the IOL 1 having less adhesion begins to come off first, leading to uneven pressing as a whole, not the intended uniform pressing. This unintended uneven pressing tends to cause undesired folding on the IOL 1 in the process of pressing the IOL 1. As a result, the IOL 1 is not correctly placed on the push axis L, raising a possibility of bad effect on subsequent pressing operations in the process of pressing by the plunger 50.

On the other hand, in the present embodiment, the whole IOL 1 is not pressed in one stroke but successively pressed from one direction by the press member 40. Specifically, when the IOL 1 sticking to the setting surface 35 outside the push axis L is pressed by the press member 40, the pressing portion 42c located at a rearmost end side firstly comes into contact with the optical part of the IOL 1. Thus, only one side of the peripheral edge of the optical part on the rear end side is pressed, and the IOL 1 sticking to the surface 35 begins to come off of the surface 35 from one direction. When the press member 40 is further pressed, the pressing portion 42b comes into contact with a center portion of the optical part to increase the contact region, thus pressing the center portion. The press member 40 is pressed furthermore, and then the pressing portion 42a located at a frontmost end side comes to contact with the optical part. As a result, the whole IOL (optical part) sticking to the surface 35 is uniformly pushed onto the axis L.

When the press member 40 is fully pressed, the whole IOL 1 is placed on the push axis L. The pressing portions 42a to 42c of the press member 40 have such a length as not to contact the plunger 50 on the axis L while the press member 40 is fully pressed against the cylinder body part 60. Accordingly, the push-out operation of the IOL 1 can be smoothly continued without removing the press member 40.

Since the IOL 1 held by the restriction member 20 is peeled off in order in one direction as mentioned above, the IOL 1 is pushed (moved) to the push axis L without being folded in the injector 100 (setting part 30) even if the IOL 1 remains sticking to the setting surface 35.

After the IOL 1 is placed on the push axis L on the setting surface 31 by the pressing operation of the press member 40, the IOL 1 is ready to be injected in the eye. At this time, the viscoelastic substance applied in the injector 100 in advance is spread into the injection part 10 by the pressing operation of the IOL 1 by the press member 40. The viscoelastic substance can be spread in a desired direction (range) because the movement direction of the IOL 1 is determined in one direction. As a result, the IOL 1 can be more favorably moved by the viscoelastic substance for injecting the IOL 1 in the eye.

Subsequently, an operator pushes out the IOL 1 in the eye. The plunger 50 is pushed along the push axis L to bring the push rod 51 frontward, so that the peripheral edge of the optical part of the IOL 1 placed on the setting surface 31 is caught by the tip 52. When the plunger 50 is further pressed from this state, the IOL 1 is gradually folded inside the injection part 10. Then, the IOL 1 is gradually injected from the front end of the injection part 10. The IOL 1 injected in the eye through an incision of an eyeball is positioned along a capsule by the restoring force of the IOL 1.

The above explanation shows an example in which the IOL 1, sticking to the setting surface 35 and arranged obliquely to the push axis L, is successively pressed by the pressing portions 42a to 42c having uniform length to move onto the axis L. Other configurations may be employed as long as the IOL sticking to the setting surface 35 can be successively peeled off from one direction. For example, relative distances between ends of pressing portions and the IOL before contacting with the ends are successively different in one direction.

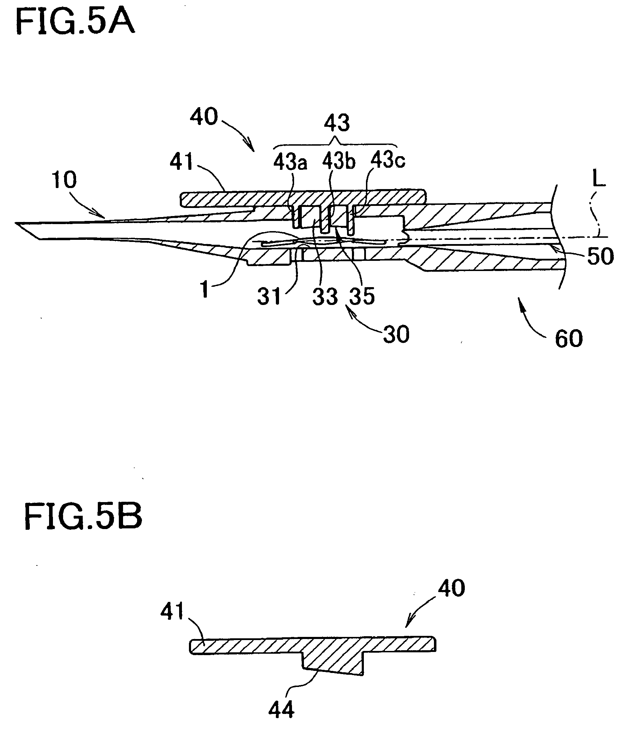

FIG. 5A shows a configuration of an injector in a second embodiment. This figure shows a state of the injector from which the restriction member 20 is detached and in which the IOL 1 is pushed on the push axis L for use. The same reference signs are applied to the same components as those of the above mentioned injector 100. In this embodiment, the setting surface 35 is formed almost parallel to the axis L so that the IOL 1 placed on the surface 35 is retained horizontally outside the axis L. On the other hand, each pressing portion 43 (43a to 43c) of the press member 40 is formed to have different length so that their distances from the IOL 1 placed outside the axis L are different from each other.

In this embodiment, the length of the pressing portions 43a to 43c are determined to be gradually shorter from the rear end side to the front end side. This configuration makes it possible that the pressing portions 43a to 43c each having different length successively come into contact with the IOL, which is retained horizontally outside the axis L, by the pressure applied by a base 41. Specifically, a contact region of an end of the pressing portion 43 with the IOL 1 is increased in order. Thereby, the IOL 1 is successively pressed on the axis L.

The above mentioned embodiment is one example of the press member 40 formed with a plurality of the pressing portions 43a to 43c. As an alternative, as shown in FIG. 5B showing a modification of the press member 40, a single pressing portion 44 having a predetermined length in the push axis L direction may be formed in the press member 40. The pressing portion 44 in this example has the length long enough to contact a predetermined region (for example, the whole optical part) of the IOL 1. According to this configuration, the contact region between the press member and the IOL can be smoothly increased, so that the IOL 1 can be favorably pressed on the axis L.

Further, the above embodiments illustrate examples that the IOL 1 is successively pressed onto the push axis L from the rear end side toward the front end side by the pressing means (the setting surface 35 and the press member 40). Alternatively, the pressing direction of the IOL 1 may be determined based on a desired region to be applied with the viscoelastic substance and others, and therefore the pressing means (the setting surface 35 and the press member 40) may be formed such that the IOL 1 is successively pressed from the front end side to the rear end side.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injection instrument (100) comprising:
an injection part (10) for injecting a folded soft intraocular lens (IOL) (1) through an incision of an eyeball, the injection part (10) having an inner wall to fold the IOL (1);
a cylinder body part (60) provided, at its front end, with the injection part (10), the cylinder body part (60) including a push member (50) movable back and forth in an axial direction for pushing the IOL (1) placed on a push axis (L);
a ceiling portion (33) provided above a setting surface (31) on which the IOL (1) is to be disposed when using, and formed with a setting surface (35) on which the IOL (1) is to be disposed when storing
a restriction member (20) attached to the cylinder body part (60) to restrict movement of the IOL (1) during storage outside the push axis (L) without applying stress to the IOL; and
pressing means (35, 40) including a press member (40) being configured with a base (41) and at least one pressing portion (42, 43, 44) movable with respect to the cylinder body part (60) through at least one through hole provided in said ceiling portion (33) to press the IOL (1) placed on said setting surface (35) outside the push axis (L), onto the setting surface (31) placed on said push axis (L), when said restriction member (20) is pulled out to release the movement restriction of the IOL (1), the pressing portion (42; 43; 44) being configured such that a contact region increases from a rear end side of the injection part (10) to a front end side or from a front end side of the injection part (10) to a rear end side so that such a combination of said press member (40) and said setting surface (35) forms pressing means for successively pressing the IOL (1).

2. The intraocular lens injection instrument (100) according to claim 1, wherein the pressing means (40) includes a plurality of the pressing portions (42a to 42c; 43a to 43c) in the axial direction of the push member (50), the pressing means being configured so that relative distances between end portions of the pressing portions (42a to 42c; 43a to 43c) and the IOL (1) before contacting with the end portions are different form each other with respect to the IOL (1) placed on the setting surface (35) to successively increase the contact region of the pressing portions (42a to 42c; 43a to 43c) to be brought into contact with the IOL (1).

3. The intraocular lens injection instrument (100) according to claim 2,
wherein the pressing portions (42a to 42c; 43a to 43c) are formed in positions corresponding to at least a peripheral edge and a center of an optical part of the IOL (1) held by the restriction member (20).

4. The intraocular lens injection instrument (100) according to any one of claims 1 to 3,
wherein the relative distances between the pressing portions (42; 43) and the IOL (1) before contacting with the pressing portions are determined to be gradually increased from a rear end side to a front end side of the cylinder body part (60) with respect to the IOL (1) placed on the setting surface (35) to successively increase the contact region of the pressing portions from the rear end side to the front end side.

## Patentansprüche

1. Ein Intraokularlinsen-Injektionsinstrument (100), das aufweist:
einen Injektionsteil (10) zum Injizieren einer gefalteten weichen Intraokularlinse (IOL) (1) durch eine Inzision an einem Augapfel, wobei in der Injektionsteil (10) eine innere Wand aufweist, um die IOL (1) zu falten;
einen Zylinderkörperteil (60), der an seinem vorderen Ende mit dem Injektionsteil (10) versehen ist, wobei der Zylinderkörperteil (60) ein Schiebebauteil (50) beinhaltet, der in einer axialen Richtung vor und zurück bewegbar ist, um die an einer Schiebeachse (L) angeordnete IOL (1) zu schieben;
einen Deckenabschnitt (33), der über einer Absetzoberfläche (31) vorgesehen ist, auf dem die IOL (1) bei Verwendung anzuordnen ist, und mit einer Absetzoberfläche (35) ausgebildet ist, auf dem die IOL (1) bei Lagerung anzuordnen ist,
ein Beschränkungsbauteil (20), dass an dem Zylinderkörperteil (60) befestigt ist, um die Bewegung der IOL (1) während der Lagerung außerhalb der Schiebeachse (L) ohne Last auf die IOL auszuüben zu beschränken; und
ein Drückmittel (35, 40), das ein Drückbauteil (40) beinhaltet, dass mit einer Basis (41) und mindestens einem drückenden Abschnitt (42, 43, 44), der bezüglich des Zylinderkörperteils (60) durch mindestens ein in dem Deckenabschnitt (33) vorgesehenen Durchgangsloch bewegbar ist, um die auf der Absetzoberfläche (35) außerhalb der Schiebeachse (L) angeordnete IOL (1) auf die auf der Schiebeachse (L) angeordnete Absetzoberfläche (31) zu drücken, wenn das Beschränkungsbauteil (20) herausgezogen wird, um die Bewegungsbeschränkung der IOL (1) freizugeben, eingerichtet ist, wobei der schiebende Abschnitt (42, 43, 44) so eingerichtet ist, dass ein Kontaktbereich von einer hinteren Endseite des Injektionsteils (10) zu einer vorderen Endseite oder von einer vorderen Endseiten des Injektionsteils (10) zu einer hinteren Endseite zunimmt, so dass solch eine Kombination aus Druckbauteil (40) und Absetzoberfläche (35) ein Drückmittel bilden, um die IOL (1) nach und nach zu drücken.

2. Das Intraokularlinsen-Injektionsinstrument (100) gemäß Anspruch 1,
wobei das Drückmittel (40) eine Vielzahl Drückabschnitte (42a-42c; 43a-43c) in der axialen Richtung des Schiebebauteils (50) beinhaltet, wobei das Drückmittel so eingerichtet ist, das relative Abstände zwischen den Endabschnitten der drückenden Abschnitte (42a-42c; 43a-43c) und der IOL (1) vor dem Kontakt mit den Endabschnitten bezüglich der auf der Absetzoberfläche (35) positioniert IOL (1) voneinander unterschiedlich sind, um den Kontaktbereich der drückenden Abschnitte (42a-42c; 43a-43c) der mit der IOL (1) in Kontakt zu bringen ist, bevor die Endabschnitte berührt werden, Schritt für Schritt zu vergrößern.

3. Das Intraokularlinsen-Injektionsinstrument (100) gemäß Anspruch 2,
wobei die drückenden Abschnitte (42a-42c; 43a-43c) in einer Position ausgebildet sind, die mindestens einer peripheren Kante und einem Zentrum eines optischen Teils der IOL (1), dass von dem Beschränkungsbauteil (20) gehalten wird, entsprechen.

4. Das Intraokularlinsen-Injektionsinstrument (100) gemäß einem der Ansprüche 1-3,
wobei die relativen Abstände zwischen dem drückenden Abschnitten (42; 43) und der IOL (1) vor dem Kontakt mit dem drückenden Abschnitten bestimmt sind, von einer hinteren Endseite zu einer vorderen Endseite des Zylinderkörperteils (60) bezüglich der auf der Absetzoberfläche angeordneten IOL (1) graduell zuzunehmen, um die Kontaktregion der pressenden Abschnitte von der hinteren Endseite zu der vorderen Endseite nach und nach zu vergrößern.

## Revendications

1. Instrument d'injection de lentille intra-oculaire (100) comprenant:
une partie d'injection (10) pour injecter une lentille intra-oculaire souple pliée (IOL) (1) à travers une incision d'un globe oculaire, la partie d'injection (10) ayant une paroi interne afin de plier la IOL (1) ;
une partie de corps de cylindre (60) prévue, au niveau de son extrémité avant, avec la partie d'injection (10), la partie de corps de cylindre (60) comprenant un corps de poussée (50) mobile selon un mouvement de va-et-vient dans une direction axiale pour pousser la IOL (1) placée sur un axe de poussée (L);
une partie de plafond (33) prévue au-dessus d'une surface de pose (31) sur laquelle la IOL (1) doit être disposée lors de l'utilisation, et formée avec une surface de pose (35) sur laquelle la IOL (1) doit être disposée, lorsqu'elle est stockée,
un élément de restriction (20) fixé sur la partie de corps de cylindre (60) pour limiter le déplacement de la IOL (1) pendant le stockage à l'extérieur de l'axe de poussée (L) sans appliquer de contrainte sur la IOL; et
des moyens de pression (35, 40) comprenant un élément de pressage (40) qui est configuré avec une base (41) et au moins une partie de pression (42, 43, 44) mobile par rapport à la partie de corps de cylindre (60) à travers au moins un trou débouchant prévu dans ladite partie de plafond (33) pour comprimer la IOL (1) placée sur ladite surface de pose (35) à l'extérieur de l'axe de poussée (L), sur la surface de pose (31) placée sur ledit axe de poussée (L), lorsque ledit élément de limitation (20) est retiré pour supprimer la limitation de déplacement de la IOL (1), la partie de pression (42; 43; 44) étant configurée de sorte qu'une région de contact augmente à partir d'un côté d'extrémité arrière de la partie d'injection (10) jusqu'à un côté d'extrémité avant ou à partir d'un côté extrémité avant de la partie d'injection (10) jusqu'à un côté d'extrémité arrière de sorte qu'une combinaison dudit élément de pressage (40) et de ladite surface de pose (35) forme des moyens de pression pour comprimer successivement la IOL (1).

2. Instrument d'injection de lentille intra-oculaire (100) selon la revendication 1, dans lequel le moyen de pression (40) comprend une pluralité de parties de pression (42a à 42c; 43a à 43c) dans la direction axiale de l'élément de poussée (50), le moyen de pression étant configuré de sorte que les distances relatives entre les parties d'extrémité des parties de pression (42a à 42c; 43a à 43c) et la IOL (1), avant d'être en contact avec les parties d'extrémité, sont différentes les unes des autres par rapport à la IOL (1) placée sur la surface de pose (35) pour augmenter successivement la région de contact des parties de pression (42a à 42c; 43a à 43c) pour être amenée en contact avec la IOL (1).

3. Instrument d'injection de lentille intra-oculaire (100) selon la revendication 2,
dans lequel les parties de pression (42a à 42c; 43a à 43c) sont formées dans des positions correspondant à au moins un bord périphérique et un centre d'une partie optique de la IOL (1) maintenue par l'élément de restriction (20).

4. Instrument d'injection de lentille intra-oculaire (100) selon l'une quelconque des revendications 1 à 3,
dans lequel les distances relatives entre les parties de pression (42; 43) et la IOL (1) avant d'être en contact avec les parties de pression, sont déterminées pour être progressivement augmentées à partir d'un côté d'extrémité arrière jusqu'à un côté d'extrémité avant de la partie de corps de cylindre (60) par rapport la IOL (1) placée sur la surface de pose (35) pour augmenter successivement la région de contact des parties de pression du côté d'extrémité arrière au côté d'extrémité avant.
